# EUROPEAN PATENT SPECIFICATION

(11) **EP 3 919 669 B1**
(45) Date of publication and mention of the grant of the patent: **20.12.2023**
(21) Application number: 21174436.2
(22) Date of filing: 18.05.2021
(51) Int. Cl.: D06F 58/02, D06F 35/00, D06F 58/45, D06F 58/22, D06F 58/24, D06F 103/10, D06F 103/12, D06F 103/24, D06F 25/00, D06F 58/20, D06F 101/20, D06F 103/38

(54) **LAUNDRY TREATMENT APPARATUS AND CONTROL METHOD THEREOF**
WÄSCHEBEHANDLUNGSVORRICHTUNG UND STEUERUNGSVERFAHREN DAFÜR
APPAREIL DE TRAITEMENT DE LINGE ET SON PROCÉDÉ DE COMMANDE

(30) Priority: 01.06.2020 KR 20200065745
(43) Date of publication of application: 08.12.2021
(73) Proprietor: LG Electronics Inc., Seoul 07336 (KR)
(72) Inventor: YOON, Juhan, Seoul 08592 (KR); AHN, Ingeun, Seoul 08592 (KR)
(74) Representative: Vossius & Partner Patentanwälte Rechtsanwälte mbB

(56) References cited:
- EP-A1- 3 190 225
- KR-A- 20070 053 045
- KR-A- 20190 012 838
- KR-B1- 101 467 494
- US-A1- 2005 066 538

## Description

This present application claims the benefit of priority to Korean Patent Application No. 10-2020-0065745, entitled "LAUNDRY TREATMENT APPARATUS AND CONTROL METHOD THEREOF," filed on June 1, 2020, in the Korean Intellectual Property Office.

The present disclosure relates to a laundry treatment apparatus and a control method thereof and, more particularly, to a laundry treatment apparatus having a function of drying clothes or bedclothes using high-temperature dry air and a control method thereof.

A laundry treatment apparatus refers to an apparatus used at home or at a laundry, to wash or dry clothes or bedclothes, or to remove wrinkles and creases from the clothes or bedclothes. That is, the laundry treatment apparatus refers to all types of apparatuses for managing or processing clothes. The laundry treatment apparatus includes a washing machine, a drying machine, a washing-and-drying machine, and the like.

Among these, the drying machine is a machine configured to evaporate moisture in a target object such as clothes or bedclothes inserted into the drum (or tub) by supplying heated air to the target object.

As the moisture is evaporated from the target object, the air discharged from the drum may have a relatively high temperature and a relatively high humidity. The high-temperature and high-humidity air discharged from the drum flows to a circulation flow path, which is provided in the drying machine, and the moisture in the air is removed at the circulation flow path. Thereafter, the moisture-removed air is heated, and then flows back to the drum.

Document US 2005/066538 A1 discloses a laundry treatment apparatus having a drum and an air circulation flow path for drying the clothes in the drum, wherein a heat exchanger is placed in the air circulation flow path and an UV sterilizer is installed in the laundry treatment apparatus so as to irradiate UV light to the heat exchanger.

With regard to such a laundry treatment apparatus having the function of drying clothes or bedclothes, Korean Patent Application Publication No. 10-2019-0128464 (hereinafter referred to as "related art 1") discloses a laundry treatment apparatus.

Specifically, the laundry treatment apparatus of related art 1 includes: a drum for containing clothing; a circulation flow path for forming a path which allows air discharged from a front opening of the drum to be introduced into a rear opening of the drum after heat exchange; and a base cabinet disposed at a lower portion of the drum to provide a space where various components are mounted.

According to the laundry treatment apparatus of related art 1, the circulation flow path is implemented in a simple structure by optimizing the design of the circulation flow path and the layout of each component. However, the invention of related art 1 cannot appropriately deal with a situation in which the circulation flow path is contaminated due to mold or bacteria growing therein.

In particular, the humidity in the circulation flow path becomes relatively high in the process of heat exchange of the air, and a relatively large amount of foreign substances such as lint are introduced into the circulation flow path. Considering these characteristics of the circulation flow path, if such contamination is not appropriately prevented, the laundry treatment apparatus may be easily contaminated, which may lead to serious complaints from users.

In addition, Korean Patent Application Publication No. 10-2015-0120054 (hereinafter referred to as "related art 2") discloses a dryer for clothes.

Specifically, related art 2 discloses a lint filter assembly for removing lint from the air discharged from the drying drum, and a lint filter cleaning device for removing lint attached to the lint filter assembly.

However, related art 2 only considers preventing degradation of efficiency of the dryer for clothes by appropriately removing lint from a circulation flow path of the air, and does not consider dealing with a situation in which the circulation flow path is contaminated due to mold or bacteria growing therein.

As described above, existing laundry treatment apparatuses for drying clothes or bedclothes using high-temperature dry air have hygiene-related issues, and these issues should be properly overcome.

The invention is specified by the independent claim. Preferred embodiments are defined in the dependent claims. The present disclosure is directed to providing a laundry treatment apparatus capable of resolving the hygiene-related issues, and a control method thereof.

Specifically, an aspect of the present invention is directed to providing a laundry treatment apparatus and a control method thereof characterized by preventing a hygiene-related problem from arising due to mold or bacteria growing in a circulation flow path for the air.

Another aspect of the present invention is directed to providing a laundry treatment apparatus and a control method thereof wherein a sterilization process for the circulation flow path and an air circulation process through the circulation flow path may be smoothly performed without interrupting each other and without functional degradation.

Yet another aspect of the present invention is directed to providing a laundry treatment apparatus and a control method thereof wherein a sterilization process for the circulation flow path may be performed in the most appropriate way according to the current state and situation of the laundry treatment apparatus.

A laundry treatment apparatus and the control method thereof according to an aspect of the present invention include a heat exchanger installed in a circulation flow path may be UV-sterilized such that growth of mold or bacteria in the circulation flow path is prevented. In detail, as UV light is irradiated to the heat exchanger from a UV sterilizer installed in the circulation flow path, the heat exchanger may be UV-sterilized.

Here, the laundry treatment apparatus and the control method thereof according to an aspect of the present invention foresee that the UV light is irradiated from the UV sterilizer towards a front surface of an evaporator of the heat exchanger.

In addition, the laundry treatment apparatus and the control method thereof according to an aspect of the present invention are characterized in that the UV light may be irradiated from the UV sterilizer through an LED module.

In addition, the laundry treatment apparatus and the control method thereof according to an aspect of the present invention are characterized in that the UV sterilizer may be installed in such a way that direct collision between the air flowing through the circulation flow path and the UV sterilizer is minimized, so as to prevent degradation of a heat exchange function and a sterilization function. Specifically, the UV sterilizer may be installed at a depressed portion of a cover frame covering an upper surface of the circulation flow path, such that direct collision between the air flowing through the circulation flow path and the UV sterilizer is minimized.

In addition, the laundry treatment apparatus and the control method thereof according to an aspect of the present invention are characterized in that the front surface of the evaporator may be cleaned in the process of circulation of the air.

Here, the laundry treatment apparatus and the control method thereof according to an aspect of the present invention are characterized in that the UV sterilizer may be installed on a first inclined surface of the cover frame, and the front surface of the evaporator may be cleaned as cleaning water runs down to the front surface of the evaporator along a second inclined surface of the cover frame.

In addition, the laundry treatment apparatus and the control method thereof according to an aspect of the present invention are characterized in that operation of the UV sterilizer may be periodically started and stopped according to a predetermined duty ratio.

In addition, the laundry treatment apparatus and the control method thereof according to an aspect of the present invention are characterized in that the operation of the UV sterilizer may be controlled according to a current state and situation of the laundry treatment apparatus. Specifically, the laundry treatment apparatus may detect a current operation mode of the laundry treatment apparatus, and operate the UV sterilizer when the laundry treatment apparatus is switched to a predetermined operation mode.

In addition, the laundry treatment apparatus and the control method thereof according to an aspect of the present invention are characterized in that a current state of the drum may be detected through a sensor, and when the current state of the drum reaches a predetermined state, the UV sterilizer may be operated.

Here, the laundry treatment apparatus and the control method thereof according to an aspect of the present invention are characterized in that a water content percentage in the drum may be estimated through an electrode sensor, and when the water content percentage reaches a predetermined value, the UV sterilizer may be operated.

In addition, the laundry treatment apparatus and the control method thereof according to an aspect of the present disclosure are characterized in that the UV sterilizer may be operated when a predetermined time period has passed from a time point at which the laundry treatment apparatus was switched to a standby mode.

In addition, the laundry treatment apparatus and the control method thereof according to an aspect of the present disclosure are characterized in that the UV sterilizer may be operated when an operation signal is inputted by a user.

Technical solutions of the present disclosure are not limited to the above-mentioned technical solutions, and other technical solutions not mentioned above will be clearly understood by those skilled in the art from the following description.

Hereinafter, effects of the laundry treatment apparatus and the control method thereof according to the present disclosure will be described.

According to at least one of the embodiments of the present disclosure, the heat exchanger may be UV-sterilized as UV light is irradiated to the heat exchanger by the UV sterilizer installed in the circulation flow path. Accordingly, mold or bacteria may be prevented from growing in the heat exchanger in the circulation flow path.

In addition, according to at least one of the embodiments of the present disclosure, the UV light may be irradiated towards the front surface of the evaporator of the heat exchanger from the UV sterilizer. Accordingly, the front surface of the evaporator, which is a portion of the UV sterilizer which foreign substances such as lint contact first and which is easy to be contaminated as mold or bacteria grows due to a relatively low temperature, may be intensively sterilized.

In addition, according to at least one of the embodiments of the present invention, as the UV light is irradiated from the UV sterilizer through the LED module, the UV sterilizer may be formed in a relatively simple structure and in relatively diverse ways, and UV light of various wavelengths may be selectively combined and used.

In addition, according to at least one of the embodiments of the present invention, the UV sterilizer may be installed at a depressed portion in the cover frame covering the upper surface of the circulation flow path, such that direct collision between the air flowing through the circulation flow path and the UV sterilizer is minimized. Accordingly, a sterilization process for the circulation flow path and an air circulation process through the circulation flow path may be smoothly performed without interrupting each other.

In addition, according to at least one of the embodiments of the present invention, as the front surface of the evaporator is cleaned in the process of circulation of the air, foreign substances such as lint and the like may be prevented from accumulating in the heat exchanger, and thus functional degradation may be prevented.

In addition, according to at least one of the embodiments of the present invention, the UV sterilizer may be installed on the first inclined surface of the cover frame, and the front surface of the evaporator may be cleaned as the cleaning water runs down to the front surface of the evaporator along the second inclined surface of the cover frame. Accordingly, the UV sterilizer and a cleaning part may be spatially separated from each other, and thus the cleaning water may be prevented from being sprayed onto the UV sterilizer.

In addition, according to at least one of the embodiments of the present invention, the operation of the UV sterilizer may be periodically started and stopped according to a predetermined duty ratio. Accordingly, intermittent and repeated sterilization stimulation may be applied to mold or bacteria, and thus, the sterilization efficiency may be further improved.

In addition, according to at least one of the embodiments of the present invention, the current operation mode of the laundry treatment apparatus may be detected, and when the laundry treatment apparatus is switched to a predetermined operation mode, the UV sterilizer may be operated. Accordingly, the sterilization process may be performed only when it is determined that the laundry treatment apparatus currently requires the sterilization process while the laundry treatment apparatus is operated.

In addition, according to at least one of the embodiments of the present invention, the current state of the drum may be detected through the sensor, and when the current state of the drum reaches a predetermined state, the UV sterilizer may be operated. Accordingly, the sterilization process may be performed only after it is determined that the laundry treatment apparatus is in a state requiring the sterilization process.

In addition, according to at least one of the embodiments of the present invention, the water content percentage in the drum may be estimated through the electrode sensor, and when the water content percentage reaches a predetermined value, the UV sterilizer may be operated. Accordingly, the sterilization process may be performed only after a drying process of the laundry treatment apparatus is practically completed.

In addition, according to at least one of the embodiments of the present invention, the UV sterilizer may be operated when a predetermined time period has passed from a time point at which the laundry treatment apparatus was switched to the standby mode. Accordingly, the sterilization process may be periodically performed even when the laundry treatment apparatus is not used for a relatively long period of time.

In addition, according to at least one of the embodiments of the present invention, the UV sterilizer may be operated when an operation signal is inputted by a user. Accordingly, users may directly select the sterilization process and have the laundry treatment apparatus perform the sterilization process as they want.

### BRIEF DESCRIPTION OF THE DRAWINGS

For the purpose of illustrating the present invention, there is shown in the drawings an exemplary embodiment, it being understood, however, that the present invention is not intended to be limited to the details shown because various modifications and structural changes may be made therein without departing from the present invention, which is defined by the appended claims.

The use of the same reference numerals or symbols in different drawings indicates similar or identical items.
FIG. 1 is a perspective view of a laundry treatment apparatus according to an embodiment of the present invention.
FIG. 2 is a diagram of main components of the laundry treatment apparatus according to an embodiment of the present invention.
FIGS. 3 and 4 are views schematically illustrating circulation of air in the laundry treatment apparatus according to an embodiment of the present invention.
FIGS. 5 and 6 are views illustrating in more detail an air circulator, a heat exchanger, and a UV sterilizer of the laundry treatment apparatus according to an embodiment of the present invention.
FIG. 7 is a view schematically illustrating a route of the air flowing through a circulation flow path of the laundry treatment apparatus according to an embodiment of the present invention.
FIG. 8 is a view illustrating in more detail a cover frame and a cleaning part of the laundry treatment apparatus according to an embodiment of the present invention.
FIGS. 9 and 10 are exemplary views illustrating a correlation between a degree of sterilization and a duty ratio in the laundry treatment apparatus according to an embodiment of the present invention.
FIG. 11 is an exemplary view illustrating a correlation between a measured value of an electrode sensor and a water content percentage in the laundry treatment apparatus according to an embodiment of the present invention.
FIG. 12 is a flow chart illustrating a control method of the laundry treatment apparatus according to an embodiment of the present invention.

### DETAILED DESCRIPTION

Advantages and features of the present invention and methods for achieving them will become apparent from the descriptions of aspects herein below with reference to the accompanying drawings. However, the present invention is not limited to the aspects disclosed herein but may be implemented in various different forms. The aspects are provided to make the description of the present invention thorough and to fully convey the scope of the present invention to those skilled in the art. It is to be noted that the scope of the present invention is defined only by the claims.

The shapes, sizes, ratios, angles, the number of elements given in the drawings are merely exemplary, and thus, the present invention is not limited to the illustrated details. Like reference numerals designate like elements throughout the specification.

In relation to describing the present invention, when the detailed description of the relevant known technology is determined to unnecessarily obscure the gist of the present invention, the detailed description may be omitted.

The terminology used herein is for the purpose of describing particular example embodiments only and is not intended to be limiting. As used herein, the singular forms "a," "an," and "the" may be intended to include the plural forms as well, unless the context clearly indicates otherwise. The terms "comprises," "comprising," "including," and "having," are inclusive and therefore specify the presence of stated features, integers, steps, operations, elements, and/or components, but do not preclude the presence or addition of one or more other features, integers, steps, operations, elements, components, and/or groups thereof. The method steps, processes, and operations described herein are not to be construed as necessarily requiring their performance in the particular order discussed or illustrated, unless specifically identified as an order of performance. It is also to be understood that additional or alternative steps may be employed.

When an element or layer is referred to as being "on," "engaged to," "connected to," or "coupled to" another element or layer, it may be directly on, engaged, connected or coupled to the other element or layer, or intervening elements or layers may be present. In contrast, when an element is referred to as being "directly on," "directly engaged to," "directly connected to," or "directly coupled to" another element or layer, there may be no intervening elements or layers present. Other words used to describe the relationship between elements should be interpreted in a like fashion (e.g., "between" versus "directly between," "adjacent" versus "directly adjacent," etc.). As used herein, the term "and/or" includes any and all combinations of one or more of the associated listed items.

Although the terms "first," "second," "third," etc. may be used herein to describe various elements, components, regions, layers and/or sections, these elements, components, regions, layers and/or sections should not be limited by these terms. These terms may be only used to distinguish one element, component, region, layer or section from another region, layer or section. Terms such as "first," "second," and other numerical terms when used herein do not imply a sequence or order unless clearly indicated by the context. Thus, a first element, component, region, layer or section discussed below could be termed a second element, component, region, layer or section without departing from the teachings of the example embodiments.

Spatially relative terms, such as "inner," "outer," "beneath," "below," "lower," "above," "upper," and the like, may be used herein for ease of description to describe one element or feature's relationship to another element(s) or feature(s) as illustrated in the figures. Spatially relative terms may be intended to encompass different orientations of the device in use or operation in addition to the orientation depicted in the figures. For example, if the device in the figures is turned over, elements described as "below" or "beneath" other elements or features would then be oriented "above" the other elements or features. Thus, the example term "below" can encompass both an orientation of above and below. The device may be otherwise oriented (rotated 90 degrees or at other orientations) and the spatially relative descriptors used herein interpreted accordingly.

The term "or" as used herein is to be interpreted as an inclusive or meaning any one or any combination. Therefore, "A, B or C" means any of the following: "A; B; C; A and B; A and C; B and C; A, B and C". An exception to this definition will occur only when a combination of elements, functions, steps or acts are in some way inherently mutually exclusive.

Hereinafter, preferable exemplary embodiments of the present invention will be described in detail referring to the attached drawings. However, description of known functions or configurations will be omitted in the following description in order to clarify the gist of the present invention.

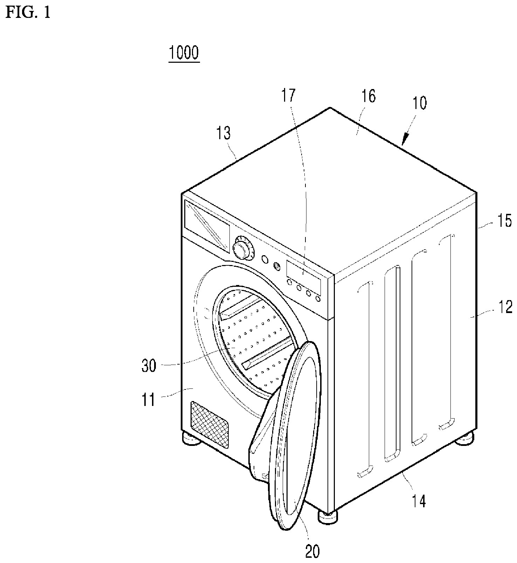

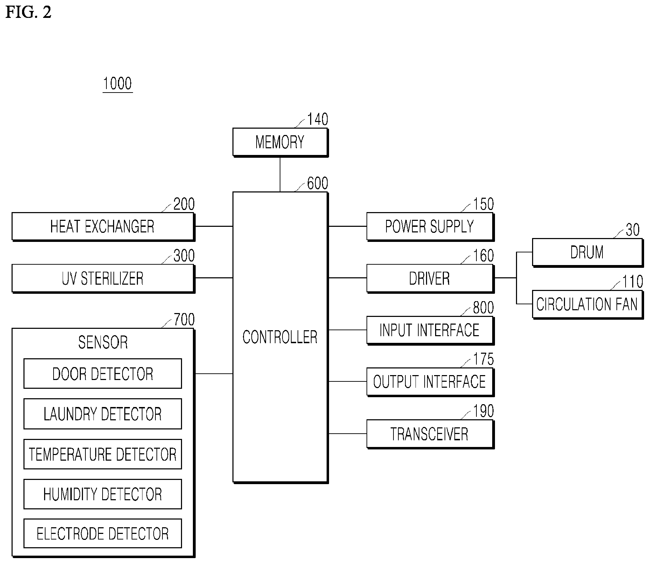

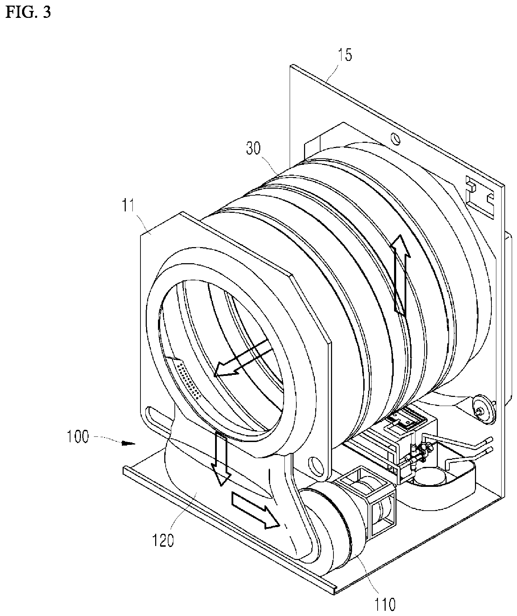

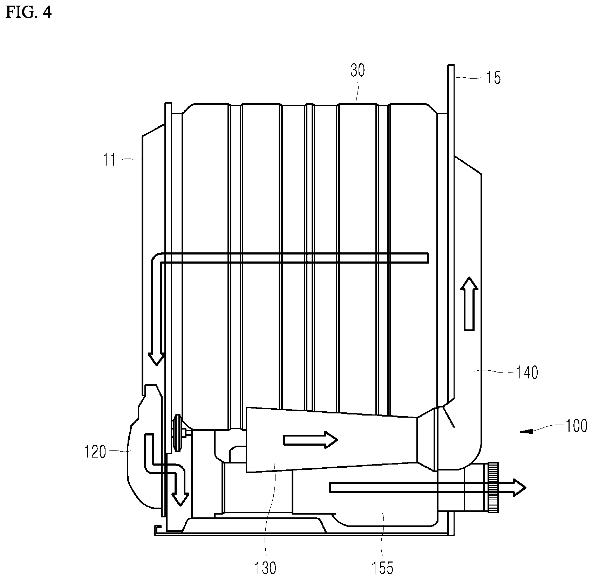

FIG. 1 is a perspective view of a laundry treatment apparatus according to an embodiment of the present invention. FIG. 2 is a diagram of main components of the laundry treatment apparatus according to an embodiment of the present invention. FIGS. 3 and 4 are views schematically illustrating circulation of air in the laundry treatment apparatus according to an embodiment of the present invention.

Hereinafter, a drying machine will be described as an example of the laundry treatment apparatus, but the laundry treatment apparatus of the present invention is not limited to the drying machine. The laundry treatment apparatus, as an apparatus configured to treat laundry (or an object to be dried) such as clothes inserted into the drum, may be a washing machine or a washing-and-drying machine.

Referring to FIGS. 1 to 4, a laundry treatment apparatus 1000 according to an embodiment of the present invention includes a cabinet 10, a drum 30 disposed inside the cabinet 10 and configured to be rotated with laundry (or fabric) received therein, a driver 160 configured to rotate the drum 30, a heat exchanger 200 configured to heat the air that is circulated from the drum 30 after drying the laundry, a circulation fan 110 configured to circulate the air in the drum 30, a suction duct 120 configured to suck the circulated air from the drum 30, and a circulation flow path 130 configured to guide a flow of the air.

The cabinet 10 may form an exterior of the laundry treatment apparatus 1000 and provide a space in which the drum 30 and other components are disposed. The cabinet 10 may be formed in the shape of a rectangular parallelepiped as a whole.

A door 20 may be disposed in a front surface of the cabinet 10, and may be rotated about a hinge provided at one side of the door 20 to open the cabinet 10. The cabinet 10 may include a front cover 11, a top plate 16, side covers 12 and 13, a rear cover 15, and a base 14.

A laundry loading hole may be formed in the front cover 11, and the door 20 may be provided in the front cover 11 to open and close the laundry loading hole. The laundry loading hole may communicate with the drum 30.

A control panel 17 may be disposed in an upper portion of the front cover 11. The control panel 17 may include a display (e.g., an LCD panel, an LED panel, etc.) on which a current operation state of the laundry treatment apparatus 1000 is displayed, an input interface 800 (e.g., a button, a dial, a touch screen, etc.) configured to receive an input of an operation command for the laundry treatment apparatus 1000 from a user, and an output interface 175 configured to output a voice guidance, a sound effect, or a warning sound for a current operation state.

The input interface 800 may include an input means installed in the control panel 17, such as at least one button, a switch, and a touch pad. The input interface 800 may receive inputs of operation settings, including an input for power on and off, an input for an operation mode, and an input for a laundry type setting. When a power-on signal is inputted and a type of laundry is selected, the input interface 800 may input data of the operation settings to a controller 600.

The output interface 175 may include a display on which information on the operation settings inputted by the input interface 800 is displayed and a current operation mode of the laundry treatment apparatus 1000 is outputted. In addition, the output interface 175 may include a speaker or a buzzer configured to output a voice guidance, or a predetermined sound effect or warning sound. The display may include a menu screen for setting and controlling operation of the laundry treatment apparatus 1000, and may output a message or a warning consisting of at least one of a letter, a number, or an image, about the operation settings and the current operation state of the laundry treatment apparatus 1000.

A memory 140 may store control data for controlling the operation of the laundry treatment apparatus 1000, data of inputted operation settings, data of an operation mode, and reference data for determining an error of the laundry treatment apparatus 1000. In addition, the memory 140 may store data that are detected or measured during an operation of the laundry treatment apparatus 1000 and data that are transmitted and received through a transceiver 190. The memory 140 may be a hardware storage device such as a ROM, a RAM, an EPROM, a flash drive, and a hard drive.

The transceiver 190 may transmit and receive data by wire or wirelessly. The transceiver 190 may transmit and receive data through a network formed within a building or within a predetermined distance, such as a home network. In addition, the transceiver 190 may be connected to an external server such as the Internet, and may communicate with a terminal having a control function.

The transceiver 190 may transmit a current operation state or a current drying state of the laundry treatment apparatus 1000, and receive a command for the laundry treatment apparatus 1000. The transceiver 190 may include a communication module, such as WiFi, WiBro, and the like, as well as through a near-field wireless communication module, such as Zigbee, Bluetooth, etc., so as to transmit and receive data.

A power supply 150 may convert supplied utility power and supply operation power. The power supply 150 may block overcurrent, and supply operation power of a predetermined amount by rectifying and smoothing the supplied power.

The drum 30 may be formed in a cylindrical shape, wherein a front surface and a back surface of the drum 30 may be open, and the front surface may communicate with the laundry loading hole. In addition, an inlet may be formed in the back surface of the drum 30 such that air can flow into the drum 30 through the inlet, and the inlet may be connected to an inflow duct 140 configured for air circulation. The inflow duct 140 may be connected to the circulation flow path 130.

The driver 160 may include a motor which is fixed to the base 14 of the cabinet 10. The motor may provide a driving force for rotating the drum 30, and may be connected to the circulation fan 110 to rotate the circulation fan 110. The air in the drum 30 may flow to the suction duct 120 due to the rotation of the circulation fan 110. The suction duct 120 may be connected to the circulation flow path 130.

When the circulation fan 110 rotates, the air may be circulated in such a manner that the air discharged from the drum 30 is guided to the suction duct 120, and then passes through the heat exchanger 200 via the circulation flow path 130, and then flows back to the drum 30 via the inflow duct 140.

The circulation flow path 130, which passes through the drum 30, may be formed in various forms. The circulation flow path 130 may be connected to the drum 30 to form a closed loop for air circulation. Here, the circulation flow path 130 may be formed in such a manner that some of the air is discharged to the outside through a discharge duct 155 as needed.

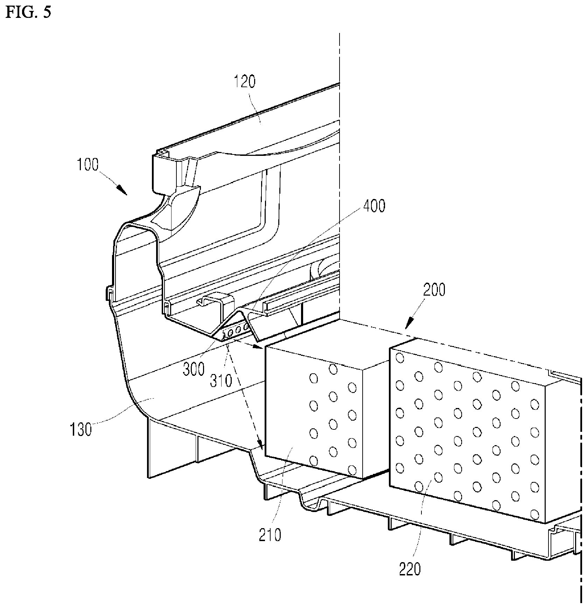

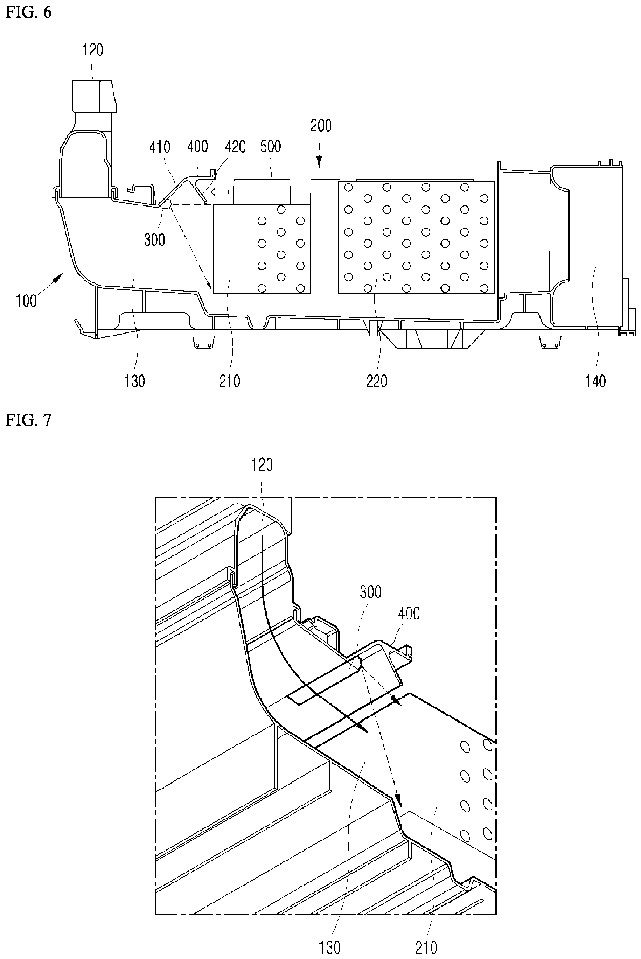

FIGS. 5 and 6 are views illustrating in more detail an air circulator, a heat exchanger, and a UV sterilizer of the laundry treatment apparatus according to an embodiment of the present invention.

As illustrated in FIGS. 5 and 6, the laundry treatment apparatus 1000 according to an embodiment of the present invention may include an air circulator 100, a heat exchanger 200, and a UV sterilizer 300.

The air circulator 100 is a component configured to guide the air discharged from the drum 30 to flow back to the drum 30, and may include the suction duct 120, the circulation flow path 130, the inflow duct 140, and the circulation fan 110, which were described in detail above.

The heat exchanger 200 is a component installed in the circulation flow path 130 of the air circulator 100 in order to perform heat exchange of the air, and may circulate a refrigerant such that a heat pump cycle is formed.

The laundry received in the drum 30 may be dried by heated air supplied to the drum 30. The air discharged from the drum 30, which contains moisture evaporated from the laundry in the drying process, may flow to the circulation flow path 130, be heated by the heat exchanger 200, and then be supplied back to the drum 30.

In detail, the heat exchanger 200 may include a compressor, an evaporator 210, a condenser 220, and an expansion valve. As the compressor, the evaporator 210, and the condenser 220 of the heat exchanger 200 are connected to a refrigerant pipe, the refrigerant may be circulated therethrough. In particular, the air that is heated as heat is exchanged between the air and the refrigerant in the condenser 220 and the evaporator 210 may be supplied to the drum 30. Here, the heat exchanger 200 may perform heat exchange of the air by using a medium other than the refrigerant, if necessary.

The evaporator 210 may absorb the heat of the air that passes through the circulation flow path 130 by exchanging heat between the refrigerant and the air that flows in the circulation flow path 130 via the suction duct 120 from the drum 30. In addition, the evaporator 210 may condense the moisture contained in the air passing through the circulation flow path 130.

In the condenser 220, heat exchange may occur between the refrigerant and the air that has passed through the evaporator 210, and the heated air may flow from the condenser 220 to the drum 30 via the inflow duct 140. In detail, the air that has passed through the evaporator 210 and thus has a relatively low temperature and a relatively low humidity may flow to the condenser 220, and when heat is exchanged between the refrigerant and the air in the condenser 220, the air may have a relatively high temperature and a relatively low humidity.

The refrigerant discharged from the condenser 220 may be recovered by the compressor after passing through the evaporator 210. The compressor may compress evaporated refrigerant and discharge the compressed refrigerant to the condenser 220. The expansion valve may expand, in the evaporator 210, the refrigerant that has been condensed in the condenser 220.

Since the air discharged from the drum 30 has a relatively high temperature and a relatively high humidity, and the temperature of the air is higher than a temperature of the refrigerant of the evaporator 210, when the air discharged from the drum 30 passes through the evaporator 210, heat is exchanged between the air and the refrigerant, and consequently, the air is condensed and cooled down. Accordingly, the air having the relatively high temperature and the relatively high humidity may be dehumidified and cooled down by the evaporator 210. Condensate water that is generated when the air is condensed may be collected by a condensate water housing, and may be discharged.

Meanwhile, in the heat exchange process described above, a large amount of moisture may come to be present in the circulation flow path 130, and in particular, the condensate water may be generated in the heat exchanger 200. That is, the circulation flow path 130 may always be in a high-humidity state. In addition, foreign substances such as a large amount of lint and the like may flow into the circulation flow path 130 from the drum 30, and the foreign substances may become attached to the heat exchanger 200.

Accordingly, the heat exchanger 200 may be contaminated by such foreign substances, and considering that the heat exchanger 200 is in a high-humidity, contaminated condition, it is highly likely that mold and bacteria will grow in the heat exchanger 200.

Accordingly, the laundry treatment apparatus 1000 according to the embodiment may be configured such that the heat exchanger 200, which is at risk of being contaminated, is UV-sterilized. For this, the UV sterilizer 300 may be installed in the circulation flow path 130 so as to irradiate UV light to the heat exchanger 200.

That is, UV light having a sterilization function may be irradiated from the UV sterilizer 300 towards the heat exchanger 200. Through this, the sterilization function may be carried out in the heat exchanger 200 by the UV light.

Here, the UV light is an invisible light with a wavelength shorter than a wavelength of visible light. Ultraviolet light with a wavelength between 200nm and 300nm or, in particular, ultraviolet light with a wavelength around 260nm, may have a relatively strong sterilization function.

As described above, in the laundry treatment apparatus 1000 according to the embodiment, the heat exchanger 200 may be UV-sterilized as the UV sterilizer 300, which is installed in the circulation flow path 130, irradiates UV light to the heat exchanger 200. Accordingly, the heat exchanger 200 in the circulation flow path 130 may be prevented from being contaminated due to growth of mold or bacteria therein.

In the laundry treatment apparatus 1000 according to the embodiment, the heat exchanger 200 may include the evaporator 210 configured to remove moisture from the air passing through the circulation flow path 130, and a condenser 220 configured to heat the air that has passed through the evaporator 210, and the UV sterilizer 300 may irradiate UV light towards a front surface of the evaporator 210.

As illustrated in FIGS. 5 and 6, the air flowing in the circulation flow path 130 may contact the front surface of the evaporator 210 first. Considering that condensate water may be formed in a certain portion of a surface of the evaporator 210 due to the function thereof, it is highly likely that foreign substances such as lint in the air flowing to the circulation flow path 130 will become attached to the front surface of the evaporator 210.

In addition, considering that a temperature of the evaporator 210 is relatively low compared to temperatures of other parts of the heat exchanger 200 due to the function thereof, it can be said that the evaporator 210 is more likely to be contaminated by mold or bacteria compared to the other parts.

Accordingly, considering that contamination of the front surface of the evaporator 210 may be the most severe, it can be said that the front surface of the evaporator 210 requires an intense sterilization.

As described above, in the laundry treatment apparatus 1000 according to the embodiment, the UV sterilizer 300 may irradiate UV light towards the front surface of the evaporator 210 of the heat exchanger 200. Accordingly, the front surface of the evaporator 210, with which the lint or other foreign substances come into contact first and which is vulnerable to the growth of mold or bacteria due to the relatively low temperature, may be intensively sterilized.

In the laundry treatment apparatus 1000 according to the embodiment, the UV sterilizer 300 may include an LED module 310 configured to irradiate the UV light by using electric energy.

Here, the LED module 310 may include a package substrate made of a ceramic, a plurality of LED chips mounted on an upper surface of the package substrate, and a fluorescent substance applied so as to cover the LED chips.

In addition, a heat dissipation pad and an electrode pad may be formed in the LED module 310, wherein the heat dissipation pad and the electrode pad may be bonded to a bonding member and a circuit pattern in a printed circuit substrate, respectively, through a soldering process or the like.

In particular, compared to a UV lamp irradiating UV light, the LED module 310 may be variously designed in relatively simple structures. In addition, when the LED module 310 selectively combines and irradiates light of various wavelengths, various sterilization functions may be carried out according to the selectively combined wavelengths.

Furthermore, since light of an LED has a relatively narrow wavelength bandwidth, light of a specific wavelength may be irradiated by the LED module 310. Accordingly, harmful ultraviolet rays (UV) or unnecessary infrared rays (IR) may not be irradiated from the LED module 310, and thus the LED module 310 may have an advantage of relatively less side effects.

As described above, in the laundry treatment apparatus 1000 according to the embodiment, the UV sterilizer 300 may irradiate UV light through the LED module 310. Accordingly, the structure of the UV sterilizer 300 may be variously implemented in relatively simple structures. In addition, UV light of various wavelengths may be selectively combined and used.

FIG. 7 is a view schematically illustrating a route of the air flowing through the circulation flow path of the laundry treatment apparatus according to an embodiment of the present invention.

The laundry treatment apparatus 1000 according to an embodiment of the present invention may further include a cover frame 400 installed in a shape that covers an upper surface of the circulation flow path 130 and is depressed upwards from an upper portion of the front surface of the evaporator 210. The UV sterilizer 300 may be installed in a depressed portion of the cover frame 400.

That is, as illustrated in FIGS. 5 to 7, the upper surface of the circulation flow path 130 and, in particular, the upper portion of the front surface of the evaporator 210, may be covered by the cover frame 400. In other words, the cover frame 400 may be formed in such a shape that a portion thereof is depressed upwards, and the UV sterilizer 300 may be installed in the depressed portion of the cover frame 400.

Accordingly, as illustrated in FIG. 7, when the air flows through the circulation flow path 130, the flow of the air may not be disrupted by the UV sterilizer 300. Consequently, as the flow of the air is not disrupted by the UV sterilizer 300 in the circulation flow path 130, the air may smoothly flow through the circulation flow path 130.

In addition, since the UV sterilizer 300 does not protrude into the flow path of the air in the circulation flow path 130, accumulation of foreign substances such as lint in the UV sterilizer 300 may be minimized.

If a large amount of foreign substances accumulates in the UV sterilizer 300, the amount of the UV light irradiated from the UV sterilizer 300 may be reduced, and as a result, the sterilization function may be impaired.

As described above, in the laundry treatment apparatus 1000 according to the embodiment, the UV sterilizer 300 may be installed in the depressed portion of the cover frame 400 which covers the upper surface of the circulation flow path 130, and thus direct collision of the air flowing through the circulation flow path 130 with the UV sterilizer 300 may be minimized. Accordingly, the sterilization process for the circulation flow path 130 and the air circulation process through the circulation flow path 130 may be smoothly carried out without interrupting each other.

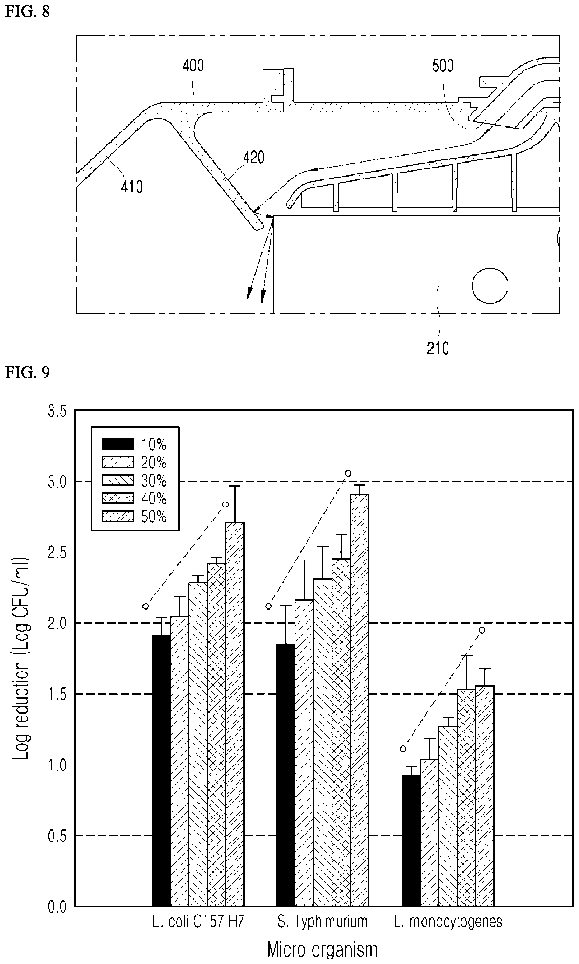

FIG. 8 is a view illustrating in more detail the cover frame and a cleaning part of the laundry treatment apparatus according to an embodiment of the present invention.

The laundry treatment apparatus 1000 according to an embodiment of the present invention may further include a cleaning part 500 configured to clean the front surface of the evaporator 210. That is, as illustrated in FIG. 8, cleaning water sprayed from the cleaning part 500 may clean the front surface of the evaporator 210.

As described above, a relatively large amount of foreign substances may become attached to the front surface of the evaporator 210. Accordingly, it is necessary to frequently clean the front surface of the evaporator 210 so as prevent the evaporator 210 from being degraded in terms of performance.

In the laundry treatment apparatus 1000 according to the embodiment, since the front surface of the evaporator 210 is cleaned in the process of the circulation of the air, functional degradation that may occur due to accumulation of foreign substances such as lint in the heat exchanger 200 may be prevented.

In the laundry treatment apparatus 1000 according to the embodiment, the cover frame 400 may include a first inclined surface 410 and a second inclined surface 420 which are coupled to each other and form a shape that is depressed upwards, wherein the UV sterilizer 300 may be installed in the first inclined surface 410, and the cleaning part 500 may spray the cleaning water onto the second inclined surface 420 such that the cleaning water runs down to the front surface of the evaporator 210 along the second inclined surface 420.

That is, as illustrated in FIG. 8, the depressed portion of the cover frame 400 may be formed of the first inclined surface 410 and the second inclined surface 420 which are coupled to each other to form a predetermined angle. In addition, the UV sterilizer 300 may be installed in the first inclined surface 410 and irradiate UV light towards the heat exchanger 200.

Meanwhile, it may be preferable that the cleaning water sprayed from the cleaning part 500 does not contact the UV sterilizer 300. Accordingly, a spraying path of the cleaning water is required to be spatially separated from the UV sterilizer 300.

By doing so, the cleaning water may be sprayed such that the cleaning water runs down to the front surface of the evaporator 210 along the second inclined surface 420 which is spaced apart from the UV sterilizer 300. That is, the cleaning water sprayed from the cleaning part 500 may collide with the second inclined surface 420 first and then run down to clean the front surface of the evaporator 210.

As described above, in the laundry treatment apparatus 1000 according to the embodiment, the UV sterilizer 300 may be installed in the first inclined surface 410 of the cover frame 400, and the cleaning part 500 may spray the cleaning water onto the second inclined surface 420 of the cover frame 400 such that the cleaning water runs down to the front surface of the evaporator 210 along the second inclined surface 420. Accordingly, the UV sterilizer 300 and the cleaning part 500 may be spatially separated from each other, and thus the cleaning water may be prevented from being sprayed onto the UV sterilizer 300.

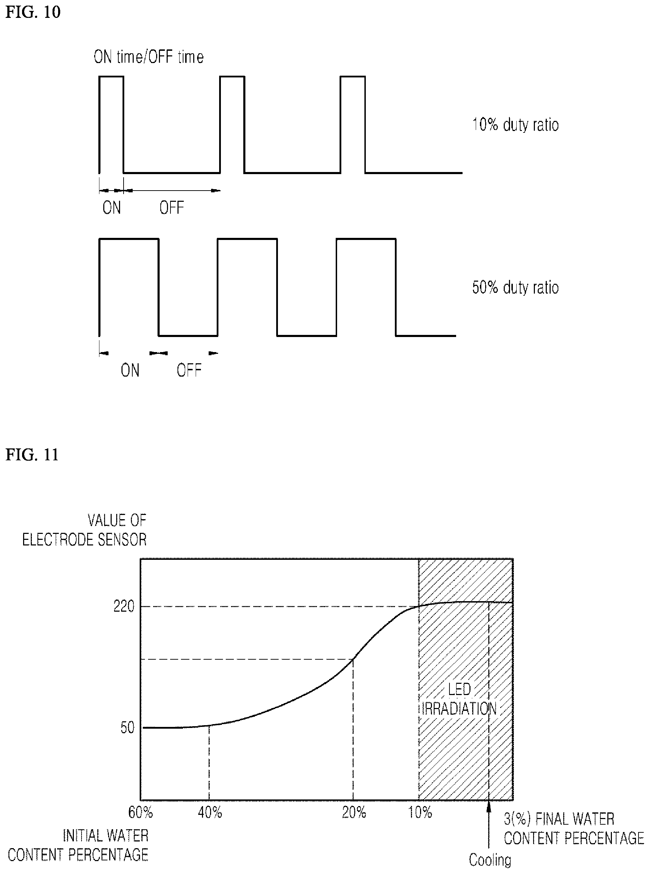

FIGS. 9 and 10 are exemplary views illustrating a correlation between a degree of sterilization and a duty ratio in the laundry treatment apparatus according to an embodiment of the present invention.

The laundry treatment apparatus 1000 according to an embodiment of the present invention may further include a controller 600 configured to control operation of the UV sterilizer 300, and the controller 600 may periodically start and stop the operation of the UV sterilizer 300 according to a predetermined duty ratio.

Here, the controller 600 may store, in the memory 140, an operation setting inputted from the input interface 800, process data transmitted from and received by the transceiver 190, and control the output interface 175 such that the operation setting and a current operation mode of the laundry treatment apparatus 1000 are outputted through the output interface 175. If there is a terminal in which an application for controlling the laundry treatment apparatus 1000 is installed and which is wirelessly connected to the laundry treatment apparatus 1000, the controller 600 may control the transceiver 190 such that data of the laundry treatment apparatus 1000 are transmitted to the terminal.

The controller 600 may control operations of the drum 30 and the circulation fan 110 through the driver 160 according to an operation setting inputted from the input interface 800, and may variably control the operations according to a sensing value of the sensor 700 which will be described below. The controller 600 may control the heat exchanger 200 such that the circulated air can be heated by the heat exchanger 200 during operation, and may control the temperature of the air supplied to the drum 30.

The controller 600 may control operation of the UV sterilizer 300. Here, the operation of the UV sterilizer 300 may include power on/off, operation time, and operation state of the UV sterilizer 300.

In particular, as illustrated in FIGS. 9 and 10, the controller 600 may adjust the duty ratio of the UV sterilizer 300 within a range from a minimum number to a maximum number of several tens. Here, the duty ratio means the proportion of on-sections to the total sections of a signal, and among the total sections, the UV light may be irradiated only in the on-sections.

When the UV sterilizer 300 does not continuously irradiate the UV light but periodically stops irradiating the UV light according to the predetermined duty ratio, the sterilization efficiency for mold and bacteria may be further improved.

For example, the LED module 310 may irradiate UV light in the form of a pulse according to a pulse width modulation (PWM) signal and the like. When the LED module 310 irradiates the UV light in the form of a pulse to the heat exchanger 200, light having a relatively high energy may be irradiated to the heat exchanger 200 for a relatively short period of time.

In addition, as the UV sterilizer 300 periodically stops irradiating the UV light, the mold or bacteria may be destroyed or damaged even by a relatively weak stimulation.

As described above, in the laundry treatment apparatus 1000 according to the embodiment, the UV sterilizer 300 does not continuously irradiate the UV light but may periodically stop irradiating the UV light according to the predetermined duty ratio. Accordingly, intermittent and repeated sterilization stimulation may be applied to the mold or bacteria, and thus, the sterilization efficiency may be further improved.

In the laundry treatment apparatus 1000 according to an embodiment of the present disclosure, the controller 600 may operate the UV sterilizer 300 when the laundry treatment apparatus 1000 is switched to a predetermined operation mode.

For example, when an operation mode in which steam is applied to the laundry in the drum 30 of the laundry treatment apparatus 1000 is in operation, the humidity of the air discharged from the drum 30 may be relatively high, and thus the efficiency of the irradiation of the UV light may be relatively low.

Accordingly, in order to improve the sterilization efficiency, the UV sterilizer 300 may be operated when a drying mode has started after the application of steam has ended in the laundry treatment apparatus 1000.

As described above, in the laundry treatment apparatus 1000 according to the embodiment, the controller 600 may detect the current operation mode of the laundry treatment apparatus 1000 and operate the UV sterilizer 300 when the laundry treatment apparatus 1000 has been switched to a predetermined operation mode. Accordingly, the sterilization process may be performed only when the laundry treatment apparatus 1000 is in a state requiring the sterilization process.

FIG. 11 is an exemplary view illustrating a correlation between a measured value of an electrode sensor and a water content percentage in the laundry treatment apparatus according to an embodiment of the present invention.

The laundry treatment apparatus 1000 according to an embodiment of the present disclosure may further include the sensor 700 installed so as to detect a current state of the drum 30, and the controller 600 may operate the UV sterilizer 300 according to the current state of the drum 30 detected by the sensor 700.

Here, the sensor 700 may include a plurality of sensors to measure a voltage or current of the laundry treatment apparatus 1000, detect a rotation speed of a motor, a temperature, and a humidity, and input the measured and detected values to the controller 600.

The sensor 700 may include a door detector, a laundry detector, a temperature detector, a humidity detector, and an electrode detector. The sensor 700 may further include a pressure sensor configured to detect a pressure of a refrigerant of the heat exchanger 200, a temperature sensor, and a speed detector configured to detect a rotation speed of the motor of the driver or a rotation speed of the drum 30.

Accordingly, through the sensor 700, it may be checked whether the laundry treatment apparatus 1000 is currently in a state requiring a sterilization process. For example, when the sensor 700 detects the temperature or humidity in the drum 30, and it is determined that the laundry treatment apparatus 1000 currently requires the sterilization process, the controller 600 may control the UV sterilizer 300 such that the UV sterilizer 300 is operated.

As described above, in the laundry treatment apparatus 1000 according to the embodiment, the UV sterilizer 300 may be operated only when the current state of the drum 30 detected by the sensor 700 reaches a predetermined state. Accordingly, the sterilization process may be performed only when it is determined that the laundry treatment apparatus 1000 currently requires the sterilization process.

In the laundry treatment apparatus 1000 according to an embodiment of the present disclosure, the sensor 700 may include an electrode sensor (which is the electrode detector in FIG. 2) installed in the drum 30, and when a water content percentage in the drum 30 that is estimated from a measured value of the electrode sensor reaches a predetermined value, the controller 600 may operate the UV sterilizer 300.

Specifically, the electrode sensor configured to detect a current state of the laundry in the drum 30 may be installed in the drum 30. The electrode sensor may be provided as two electrode sensors including an anode and a cathode spaced apart from each other by a certain distance, and the two electrode sensors may be installed so as to be exposed to the drum 30.

The electrode sensors may come into contact with the laundry in the drum 30 while the drum 30 is rotating, and accordingly, the electrode sensors may detect the current state of the laundry and, in particular, water content in the laundry (i.e., the water content percentage). According to the water content percentage detected by the electrode sensors, the controller 600 may determine whether the laundry is in a dry state.

When the laundry contacts the electrode sensors, the anode and the cathode may be brought into conduction by the moisture contained in the laundry, thus forming a closed circuit, and a value of current that flows in the circuit may vary depending on the amount of moisture contained in the laundry. Therefore, the degree of dryness of the laundry may be determined on the basis of the value of current. The value of current flowing in the circuit may also vary, since the laundry may act as a resistance against an electrode, and a value of the resistance may vary depending on the amount of moisture contained in the laundry..

As described above, the water content percentage in the drum 30 may be estimated through the electrode sensors, and when an estimated water content percentage reaches a predetermined value (for example, 10% in FIG. 11), it may be determined that the laundry is currently in a dry state in which it is appropriate to perform the sterilization process.

Accordingly, only when the water content percentage estimated through the electrode sensors reaches a predetermined value, the UV sterilizer 300 may be operated to perform the sterilization process.

As described above, in the laundry treatment apparatus 1000 according to the embodiment, the UV sterilizer 300 may be operated when the water content percentage estimated through the electrode sensors reaches a predetermined value. Accordingly, the sterilization process may be performed after a drying process has been practically completed in the laundry treatment apparatus 1000.

In the laundry treatment apparatus 1000 according to an embodiment of the present disclosure, the controller 600 may operate the UV sterilizer 300 when a predetermined time period has passed after a time point at which the laundry treatment apparatus 1000 was switched to a standby mode.

As described in detail above, when the laundry treatment apparatus 1000 is switched to a predetermined operation mode, or the drum 30 is in a predetermined state, the UV sterilizer 300 may be operated to perform the sterilization process.

However, when the laundry treatment apparatus 1000 is not used for a long period of time, the laundry treatment apparatus 1000 cannot be switched to a predetermined operation mode and the drum 30 cannot reach a predetermined state to thereby operate the UV sterilizer 300, and thus mold or bacteria may grow in the circulation flow path 130.

Accordingly, even when the laundry treatment apparatus 1000 is not used for a long period of time, the laundry treatment apparatus 1000 may be set in such a way that the sterilization process is automatically performed for the heat exchanger 200 when a predetermined time period has passed.

As described above, in the laundry treatment apparatus 1000 according to the embodiment, the UV sterilizer 300 may be operated when a predetermined time period has passed after a time point at which the laundry treatment apparatus 1000 was switched to the standby mode. Accordingly, the sterilization process may be periodically performed even when the laundry treatment apparatus 1000 is not used for a relatively long period of time.

Meanwhile, all of the predetermined duty ratio, the predetermined operation mode, the predetermined value, and the predetermined time period may be factors which are appropriately determined on the basis of experiments conducted in advance or statistical data, and may be variously set in a design stage of the laundry treatment apparatus 1000 or may be set by the user while the laundry treatment apparatus 1000 is used.

The laundry treatment apparatus 1000 according to an embodiment of the present disclosure may further include the input interface 800 to which an operation signal is inputted by the user, and the controller 600 may operate the UV sterilizer 300 when the operation signal is inputted to the input interface 800.

As described in detail above, the UV sterilizer 300 may be automatically operated when the laundry treatment apparatus 1000 is switched to a predetermined operation mode or the drum 30 is in a predetermined state. In addition, there is a possibility that the user may want to perform the sterilization process right away.

Considering that different users may have different sensitivities to the degree of contamination depending on his or her personality, it is anticipated that the frequency of performing the sterilization process may be different for each different user.

Accordingly, a user who wants to directly manipulate the laundry treatment apparatus 1000 to perform the sterilization process may input a signal to the input interface 800 for operating the UV sterilizer 300.

As described above, in the laundry treatment apparatus 1000 according to the embodiment, a user may be able to directly input an operation signal to the input interface 800 in order to operate the UV sterilizer 300. Accordingly, different users may directly operate the laundry treatment apparatus 1000 in different ways to operate the UV sterilizer 300.

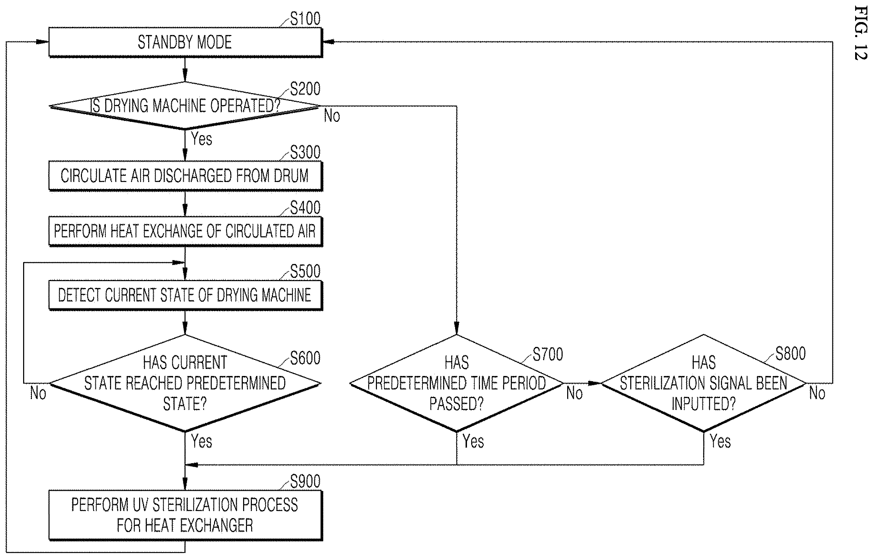

FIG. 12 is a flow chart illustrating a control method of the laundry treatment apparatus according to an embodiment of the present invention. Here, the control method of the laundry treatment apparatus according to the embodiment may include the main components of the laundry treatment apparatus 1000 described in detail above. Therefore, FIG. 12 will be described referring to FIGS. 1 to 11 together.

Firstly, the laundry treatment apparatus 1000 may be switched from the standby mode (S100) to an operation mode (S200) by an input of, for example, a predetermined operation signal.

Next, while the laundry treatment apparatus 1000 is in operation, the air may be circulated in such a way that the air discharged from the drum 30 is guided to flow back to the drum 30 (S300). Here, the air may be circulated through the suction duct 120, the circulation flow path 130, the inflow duct 140, and the circulation fan 110.

Next, while the air is circulated, heat of the air may be exchanged by the heat exchanger 200 installed in the circulation flow path 130 (S400). That is, the air discharged from the drum 30, which contains moisture evaporated from the laundry in the drying process, may flow to the circulation flow path 130, may be heated through the heat exchanger 200, and then may be supplied back to the drum 30.

Then, the UV sterilizer 300 may be controlled so as to irradiate UV light to the heat exchanger 200 (S500, S600, S700, S800, and S900). That is, the heat exchanger 200, which is at risk of being contaminated by mold or bacteria, may be UV-sterilized. For this, the UV sterilizer 300 may be installed in the circulation flow path 130 so as to irradiate the UV light to the heat exchanger 200.

The UV sterilizer 300 may be controlled in various ways depending on the current operation mode of the laundry treatment apparatus 1000 or the current state of the drum 30.

In the control method of the laundry treatment apparatus 1000 according to an embodiment of the present disclosure, the operation of the UV sterilizer 300 may be periodically started and stopped according to a predetermined duty ratio in a step of controlling the UV sterilizer 300 (in particular, in S900).

That is, as the UV sterilizer 300 does not continuously irradiate the UV light but periodically stops irradiating the UV light according to the predetermined duty ratio, the sterilization efficiency for mold and bacteria may be further improved.

In the control method of the laundry treatment apparatus 1000 according to an embodiment of the present disclosure, the step of controlling the UV sterilizer 300 may include a step of detecting a current operation mode of the laundry treatment apparatus 1000 (S500) and a step of operating the UV sterilizer 300 (S900) when the laundry treatment apparatus 1000 is switched to a predetermined operation mode (or to a predetermined state) (S600).

That is, the UV sterilizer 300 may be operated on the basis of whether the laundry treatment apparatus 1000 currently requires the sterilization process of the UV sterilizer 300. Accordingly, the sterilization process may be performed only when it is determined that the laundry treatment apparatus 1000 currently requires the sterilization process while the laundry treatment apparatus 1000 is operated.

In the control method of the laundry treatment apparatus 1000 according to an embodiment of the present disclosure, the step of controlling the UV sterilizer 300 may include a step of detecting a current state of the drum 30 through the sensor 700 (S500) and a step of operating the UV sterilizer 300 (S900) when the current state of the drum 30 detected by the sensor 700 reaches a predetermined state (S600).

That is, after it is determined, through the sensor 700, whether the laundry treatment apparatus 1000 currently requires the sterilization process, the sterilization process may be performed by the UV sterilizer 300.

In the control method of the laundry treatment apparatus 1000 according to an embodiment of the present disclosure, the step of controlling the UV sterilizer 300 may include a step of operating the UV sterilizer 300 (S900) when a predetermined time period has passed after a time point at which the laundry treatment apparatus 1000 was switched to the standby mode (S700).

That is, even when the laundry treatment apparatus 1000 is not used for a relatively long period of time, the laundry treatment apparatus 1000 may be set in such a way that the sterilization process is automatically performed for the heat exchanger 200 when a predetermined time period has passed.

In the control method of the laundry treatment apparatus 1000 according to an embodiment of the present disclosure, the step of controlling the UV sterilizer 300 may include a step of operating the UV sterilizer 300 (S900) when an operation signal is inputted by a user (S800).

That is, a user who wants to directly manipulate the laundry treatment apparatus 1000 to perform the sterilization process may input a signal to the input interface 800 for operating the UV sterilizer 300.

## Claims

1. A laundry treatment apparatus (1000) comprising:
a drum (30) rotatably installed within a cabinet (10);
a suction duct (120) to which the air discharged from the drum (30) flows;
a circulation flow path (130) connected to the suction duct (120) to guide the air to flow;
an inflow duct (140) configured to connect the circulation flow path (130) to the drum (30) such that the air flows back to the drum (30) through the inflow duct (140);
a circulation fan (110) configured to form a flow of air between the suction duct (120) and the inflow duct (140);
a heat exchanger (200) installed in the circulation flow path (130) and configured to perform heat exchange of the air; and
a UV sterilizer (300) installed in a space in the circulation flow path (130) that is formed above a front surface of the heat exchanger (200) so as to irradiate UV light to the heat exchanger (200).

2. The laundry treatment apparatus (1000) of claim 1, wherein the heat exchanger (200) comprises:
an evaporator (210) configured to remove moisture from the air passing through the circulation flow path (130); and
a condenser (220) configured to heat the air that has passed through the evaporator (210),
wherein the UV sterilizer (300) irradiates UV light to a front surface of the evaporator (210).

3. The laundry treatment apparatus (1000) of claim 2, wherein the UV sterilizer (300) comprises an LED module (310) configured to irradiate UV light by using electric energy.

4. The laundry treatment apparatus (1000) of claim 3, further comprising a cover frame (400) installed in a shape that covers an upper surface of the circulation flow path (130) and is depressed upwards from an upper portion of the front surface of the evaporator (210),
wherein the UV sterilizer (300) is installed in a depressed portion of the cover frame (400).

5. The laundry treatment apparatus (1000) of claim 4, further comprising a cleaning part (500) configured to clean the front surface of the evaporator (210).

6. The laundry treatment apparatus (1000) of claim 5, wherein
the cover frame (400) comprises a first inclined surface (410) and a second inclined surface (420) which are coupled to each other and form a shape that is depressed upwards,
the UV sterilizer (300) is installed on the first inclined surface (410), and
the cleaning part (500) sprays cleaning water onto the second inclined surface (420) such that the cleaning water runs down to the front surface of the evaporator (210) along the second inclined surface (420).

7. The laundry treatment apparatus (1000) of any one of claims 1 to 6, further comprising a controller (600) configured to control operation of the UV sterilizer (300),
wherein the operation of the UV sterilizer (300) is periodically started and stopped by the controller (600) according to a predetermined duty ratio.

8. The laundry treatment apparatus (1000) of claim 7, wherein the controller (600) operates the UV sterilizer (300) when the laundry treatment apparatus (1000) is switched to a predetermined operation mode.

9. The laundry treatment apparatus (1000) of claim 7 or 8, further comprising a sensor (700) installed so as to detect a current state of the drum (30),
wherein the controller (600) operates the UV sterilizer (300) according to the current state of the drum (30) detected by the sensor (700).

10. The laundry treatment apparatus (1000) of claim 9, wherein
the sensor (700) comprises an electrode sensor installed in the drum (30), and
the controller (600) operates the UV sterilizer (300) when a water content percentage in the drum (30), which is estimated from a measured value detected by the electrode sensor, reaches a predetermined value.

11. The laundry treatment apparatus (1000) of any one of claims 7 to 10, wherein the controller (600) operates the UV sterilizer (300) when a predetermined time period has passed after a time point at which the laundry treatment apparatus (1000) was switched to a standby mode.

12. The laundry treatment apparatus (1000) of claim 7, further comprising an input interface (800) to which an operation signal is inputted by a user,
wherein the controller (600) operates the UV sterilizer (300) when an operation signal is inputted to the input interface (800).

13. A control method of a laundry treatment apparatus (1000),
the laundry treatment apparatus (1000) comprising a drum (30), a suction duct (120), a circulation flow path (130), an inflow duct (140), a circulation fan (110), a heat exchanger (200), and a UV sterilizer (300), and
the control method comprising:
switching the laundry treatment apparatus (1000) from a standby mode to an operation mode;
circulating the air in such a way that after the air discharged from the drum (30) flows to the suction duct (120), the air is guided in the circulation flow path (130) to flow back to the drum (30) through the inflow duct (140);
performing heat exchange of the circulated air through the heat exchanger (200) installed in the circulation flow path (130); and
controlling the UV sterilizer (300) such that the UV sterilizer (300), which is installed in the circulation flow path (130) at an upper portion of a front surface of the heat exchanger (200), irradiates UV light to the heat exchanger (200).

14. The control method of the laundry treatment apparatus (1000) of claim 13, wherein in the step of controlling the UV sterilizer (300), an operation of the UV sterilizer (300) is periodically started and stopped according to a predetermined duty ratio.

15. The control method of the laundry treatment apparatus (1000) of claim 14, wherein the controlling of the UV sterilizer (300) comprises:
detecting a current operation mode of the laundry treatment apparatus (1000); and
operating the UV sterilizer (300) when the laundry treatment apparatus (1000) is switched to a predetermined operation mode.

## Patentansprüche

1. Wäschebehandlungsvorrichtung (1000), mit:
einer drehbar in einem Gehäuse (10) installierten Trommel (30);
einem Ansaugkanal (120), zu dem die von der Trommel (30) abgegebene Luft strömt;
einem mit dem Ansaugkanal (120) verbundenen Umwälzströmungspfad (130) zum Führen der Luftströmung;
einem Zustromkanal (140), der dafür konfiguriert ist, den Umwälzströmungspfad (130) mit der Trommel (30) zu verbinden, so dass die Luft über den Zustromkanal (140) zur Trommel (30) zurück strömt;
einem Umwälzgebläse (110), das dafür konfiguriert ist, einen Luftstrom zwischen dem Ansaugkanal (120) und dem Zustromkanal (140) zu bilden;
einem Wärmetauscher (200), der im Umwälzströmungspfad (130) installiert und dafür konfiguriert ist, einen Wärmeaustausch der Luft auszuführen; und
einem UV-Sterilisator (300), der in einem Raum im Umwälzströmungspfad (130) installiert ist, der über einer Vorderfläche des Wärmetauschers (200) ausgebildet ist, um UV-Licht auf den Wärmetauscher (200) einzustrahlen.

2. Wäschebehandlungsvorrichtung (1000) nach Anspruch 1, wobei der Wärmetauscher (200) aufweist:
einen Verdampfer (210), der dafür konfiguriert ist, der den Umwälzströmungspfad (130) durchströmenden Luft Feuchtigkeit zu entziehen; und
einen Kondensator (220), der dafür konfiguriert ist, die Luft, die den Verdampfer (210) durchströmt hat, zu erwärmen,
wobei der UV-Sterilisator (300) UV-Licht auf eine Vorderfläche des Verdampfers (210) einstrahlt.

3. Wäschebehandlungsvorrichtung (1000) nach Anspruch 2, wobei der UV-Sterilisator (300) ein LED-Modul (310) aufweist, das dafür konfiguriert ist, UV-Licht unter Verwendung elektrischer Energie abzustrahlen.

4. Wäschebehandlungsvorrichtung (1000) nach Anspruch 3, ferner mit einem Abdeckrahmen (400), der in einer Form installiert ist, die eine obere Fläche des Umwälzströmungspfads (130) abdeckt und bezüglich eines oberen Abschnitts der Vorderfläche des Verdampfers (210) nach oben vertieft ist,
wobei der UV-Sterilisator (300) in einem vertieften Abschnitt des Abdeckrahmens (400) installiert ist.

5. Wäschebehandlungsvorrichtung (1000) nach Anspruch 4, ferner mit einem Reinigungsteil (500), das dafür konfiguriert ist, die Vorderfläche des Verdampfers (210) zu reinigen.

6. Wäschebehandlungsvorrichtung (1000) nach Anspruch 5, wobei
der Abdeckrahmen (400) eine erste geneigte Fläche (410) und eine zweite geneigte Fläche (420) aufweist, die miteinander verbunden sind und eine nach oben vertiefte Form bilden,
der UV-Sterilisator (300) auf der ersten geneigten Fläche (410) installiert ist, und
das Reinigungsteil (500) Reinigungswasser auf die zweite geneigte Fläche (420) sprüht, so dass das Reinigungswasser entlang der zweiten geneigten Fläche (420) zur Vorderfläche des Verdampfers (210) herabläuft.

7. Wäschebehandlungsvorrichtung (1000) nach einem der Ansprüche 1 bis 6, ferner mit einer Steuereinheit (600), die dafür konfiguriert ist, den Betrieb des UV-Sterilisators (300) zu steuern,
wobei der Betrieb des UV-Sterilisators (300) durch die Steuereinheit (600) gemäß einem vorgegebenen Arbeitszyklus periodisch gestartet und gestoppt wird.

8. Wäschebehandlungsvorrichtung (1000) nach Anspruch 7, wobei die Steuereinheit (600) den UV-Sterilisator (300) aktiviert, wenn die Wäschebehandlungsvorrichtung (1000) in einen vorgegebenen Betriebsmodus geschaltet wird.

9. Wäschebehandlungsvorrichtung (1000) nach Anspruch 7 oder 8, ferner mit einem Sensor (700), der derart installiert ist, dass er einen aktuellen Zustand der Trommel (30) erfasst,
wobei die Steuereinheit (600) den UV-Sterilisator (300) gemäß dem durch den Sensor (700) erfassten aktuellen Zustand der Trommel (30) aktiviert.

10. Wäschebehandlungsvorrichtung (1000) nach Anspruch 9, wobei
der Sensor (700) einen in der Trommel (30) installierten Elektrodensensor aufweist, und
die Steuereinheit (600) den UV-Sterilisator (300) aktiviert, wenn ein prozentualer Wassergehalt in der Trommel (30), der aus einem durch den Elektrodensensor erfassten Messwert geschätzt wird, einen vorgegebenen Wert erreicht.

11. Wäschebehandlungsvorrichtung (1000) nach einem der Ansprüche 7 bis 10, wobei die Steuereinheit (600) den UV-Sterilisator (300) aktiviert, wenn eine vorgegebene Zeitdauer nach einem Zeitpunkt verstrichen ist, zu dem die Wäschebehandlungsvorrichtung (1000) in einen Bereitschaftsmodus geschaltet wurde.

12. Wäschebehandlungsvorrichtung (1000) nach Anspruch 7, ferner mit einer Eingabeschnittstelle (800), in die ein Betriebssignal durch einen Benutzer eingegeben wird,
wobei die Steuereinheit (600) den UV-Sterilisator (300) aktiviert, wenn ein Betriebssignal in die Eingabeschnittstelle (800) eingegeben wird.

13. Steuerverfahren für eine Wäschebehandlungsvorrichtung (1000),
wobei die Wäschebehandlungsvorrichtung (1000) eine Trommel (30), einen Ansaugkanal (120), einen Umwälzströmungspfad (130), einen Zustromkanal (140), ein Umwälzgebläse (110), einen Wärmetauscher (200) und einen UV-Sterilisator (300) aufweist, und
wobei das Steuerverfahren die Schritte aufweist:
Umschalten der Wäschebehandlungsvorrichtung (1000) von einem Bereitschaftsmodus in einen Betriebsmodus;
Umwälzen der Luft derart, dass die Luft, nachdem die von der Trommel (30) abgegebene Luft zum Ansaugkanal (120) strömt, in den Umwälzströmungspfad (130) geleitet wird, so dass sie über den Zustromkanal (140) zur Trommel (30) zurück strömt;
Ausführen eines Wärmeaustauschs der umgewälzten Luft durch den im Umwälzströmungspfad (130) installierten Wärmetauscher (200); und
Steuern des UV-Sterilisators (300) derart, dass der UV-Sterilisator (300), der in dem Umwälzströmungspfad (130) an einem oberen Abschnitt einer Vorderfläche des Wärmetauschers (200) installiert ist, UV-Licht auf den Wärmetauscher (200) einstrahlt.

14. Steuerverfahren für eine Wäschebehandlungsvorrichtung (1000) nach Anspruch 13, wobei im Schritt zum Steuern des UV-Sterilisators (300) ein Betrieb des UV-Sterilisators (300) gemäß einem vorgegebenen Arbeitszyklus periodisch gestartet und gestoppt wird.

15. Steuerverfahren für eine Wäschebehandlungsvorrichtung (1000) nach Anspruch 14, wobei das Steuern des UV-Sterilisators (300) aufweist:
Erfassen eines aktuellen Betriebsmodus der Wäschebehandlungsvorrichtung (1000); und
Aktivieren des UV-Sterilisators (300), wenn die Wäschebehandlungsvorrichtung (1000) in einen vorgegebenen Betriebsmodus geschaltet ist.

## Revendications

1. Appareil de traitement du linge (1000) comprenant :
un tambour (30) installé de manière rotative à l'intérieur d'une carrosserie (10) ;
un conduit d'aspiration (120) vers lequel l'air évacué à partir du tambour (30) s'écoule ;
un trajet d'écoulement de circulation (130) relié au conduit d'aspiration (120) pour guider l'écoulement de l'air ;
un conduit d'entrée (140) configuré pour relier le trajet d'écoulement de circulation (130) au tambour (30) de sorte que l'air reflue vers le tambour (30) à travers le conduit d'entrée (140) ;
un ventilateur de circulation (110) configuré pour former un écoulement d'air entre le conduit d'aspiration (120) et le conduit d'entrée (140) ;
un échangeur de chaleur (200) installé dans le trajet d'écoulement de circulation (130) et configuré pour réaliser un échange thermique de l'air ; et
un stérilisateur UV (300) installé dans un espace dans le trajet d'écoulement de circulation (130) qui est formé au-dessus d'une surface avant de l'échangeur de chaleur (200) de manière à irradier de la lumière UV vers l'échangeur de chaleur (200).

2. Appareil de traitement du linge (1000) selon la revendication 1, dans lequel l'échangeur de chaleur (200) comprend :
un évaporateur (210) configuré pour éliminer de l'humidité de l'air passant à travers le trajet d'écoulement de circulation (130) ; et
un condenseur (220) configuré pour chauffer l'air qui est passé à travers l'évaporateur (210),
dans lequel le stérilisateur UV (300) irradie de la lumière UV vers une surface avant de l'évaporateur (210).

3. Appareil de traitement du linge (1000) selon la revendication 2, dans lequel le stérilisateur UV (300) comprend un module à LED (310) configuré pour irradier de la lumière UV en utilisant de l'énergie électrique.

4. Appareil de traitement du linge (1000) selon la revendication 3, comprenant en outre un cadre de recouvrement (400) installé sous une forme qui recouvre une surface supérieure du trajet d'écoulement de circulation (130) et qui est enfoncé vers le haut à partir d'une partie supérieure de la surface avant de l'évaporateur (210),
dans lequel le stérilisateur UV (300) est installé dans une portion enfoncée du cadre de recouvrement (400).

5. Appareil de traitement du linge (1000) selon la revendication 4, comprenant en outre une partie de nettoyage (500) configurée pour nettoyer la surface avant de l'évaporateur (210).

6. Appareil de traitement du linge (1000) selon la revendication 5, dans lequel
le cadre de recouvrement (400) comprend une première surface inclinée (410) et une deuxième surface inclinée (420) qui sont accouplées l'une à l'autre et forment une forme qui est enfoncée vers le haut,
le stérilisateur UV (300) est installé sur la première surface inclinée (410), et
la partie de nettoyage (500) pulvérise de l'eau de nettoyage sur la deuxième surface inclinée (420) de sorte que l'eau de nettoyage coule vers la surface avant de l'évaporateur (210) le long de la deuxième surface inclinée (420).

7. Appareil de traitement du linge (1000) selon l'une quelconque des revendications 1 à 6, comprenant en outre un dispositif de commande (600) configuré pour commander le fonctionnement du stérilisateur UV (300),
dans lequel le fonctionnement du stérilisateur UV (300) est périodiquement démarré et arrêté par le dispositif de commande (600) selon un rapport cyclique prédéterminé.

8. Appareil de traitement du linge (1000) selon la revendication 7, dans lequel le dispositif de commande (600) fait fonctionner le stérilisateur UV (300) lorsque l'appareil de traitement du linge (1000) est commuté à un mode de fonctionnement prédéterminé.

9. Appareil de traitement du linge (1000) selon la revendication 7 ou 8, comprenant en outre un capteur (700) installé de manière à détecter un état actuel du tambour (30),
dans lequel le dispositif de commande (600) fait fonctionner le stérilisateur UV (300) selon l'état actuel du tambour (30) détecté par le capteur (700).

10. Appareil de traitement du linge (1000) selon la revendication 9, dans lequel
le capteur (700) comprend un capteur d'électrode installé dans le tambour (30), et
le dispositif de commande (600) fait fonctionner le stérilisateur UV (300) lorsqu'un pourcentage de teneur en eau dans le tambour (30), qui est estimé à partir d'une valeur mesurée détectée par le capteur d'électrode, atteint une valeur prédéterminée.

11. Appareil de traitement du linge (1000) selon l'une quelconque des revendications 7 à 10, dans lequel le dispositif de commande (600) fait fonctionner le stérilisateur UV (300) lorsqu'une période de temps prédéterminée s'est écoulée après un point de temps auquel l'appareil de traitement du linge (1000) a été commuté à un mode veille.

12. Appareil de traitement du linge (1000) selon la revendication 7, comprenant en outre une interface d'entrée (800) sur laquelle un signal de fonctionnement est entré par un utilisateur,
dans lequel le dispositif de commande (600) fait fonctionner le stérilisateur UV (300) lorsqu'un signal de fonctionnement est entré sur l'interface d'entrée (800).

13. Procédé de commande d'un appareil de traitement du linge (1000),
l'appareil de traitement du linge (1000) comprenant un tambour (30), un conduit d'aspiration (120), un trajet d'écoulement de circulation (130), un conduit d'entrée (140), un ventilateur de circulation (110), un échangeur de chaleur (200) et un stérilisateur UV (300), et
le procédé de commande comprenant :
une commutation de l'appareil de traitement du linge (1000) d'un mode veille à un mode de fonctionnement ;
une action de faire circuler de l'air de telle sorte qu'après que l'air évacué du tambour (30) s'écoule vers le conduit d'aspiration (120), l'air soit guidé dans le trajet d'écoulement de circulation (130) pour refluer vers le tambour (30) par le conduit d'entrée (140) ;
une réalisation d'un échange thermique de l'air circulant à travers l'échangeur de chaleur (200) installé dans le trajet d'écoulement de circulation (130) ; et
une commande du stérilisateur UV (300) de sorte que le stérilisateur UV (300), qui est installé dans le trajet d'écoulement de circulation (130) au niveau d'une portion supérieure d'une surface avant de l'échangeur de chaleur (200), irradie de la lumière UV vers l'échangeur de chaleur (200).

14. Procédé de commande de l'appareil de traitement du linge (1000) selon la revendication 13, dans lequel, à l'étape de commande du stérilisateur UV (300), un fonctionnement du stérilisateur UV (300) est périodiquement démarré et arrêté selon un rapport cyclique prédéterminé.

15. Procédé de commande de l'appareil de traitement du linge (1000) selon la revendication 14, dans lequel la commande du stérilisateur UV (300) comprend :
une détection d'un mode de fonctionnement actuel de l'appareil de traitement du linge (1000) ; et
une action de faire fonctionner le stérilisateur UV (300) lorsque l'appareil de traitement du linge (1000) est commuté à un mode de fonctionnement prédéterminé.
